# EUROPÄISCHE PATENTSCHRIFT

(11) **EP 1 902 317 B1**
(45) Veröffentlichungstag und Bekanntmachung des Hinweises auf die Patenterteilung: **12.09.2012**
(21) Anmeldenummer: 06754665.5
(22) Anmeldetag: 04.07.2006
(51) Int. Cl.: B01J 20/32, B01D 15/00, G01N 33/543, G01N 33/68, G01N 33/58

(54) **VERFAHREN ZUR SELEKTIVEN BESTIMMUNG PATHOLOGISCHER PROTEINABLAGERUNGEN**
METHOD FOR THE SELECTIVE DETECTION OF PATHOLOGICAL PROTEIN DEPOSITIONS
PROCEDE DE DETERMINATION SELECTIVE DE DEPOTS PROTEIQUES PATHOLOGIQUES

(30) Priorität: 04.07.2005 DE 102005031429
(43) Veröffentlichungstag der Anmeldung: 26.03.2008
(73) Patentinhaber: Heinrich-Heine-Universität Düsseldorf, 40225 Düsseldorf (DE)
(72) Erfinder: BIRKMANN, Eva, 41352 Korschenbroich (DE); RIESNER, Detlev, 40547 Düsseldorf (DE); WEINMANN, Nicole, 26603 Aurich (DE); SCHÄFER, Oliver, 45239 Essen (DE)
(74) Vertreter: von Kreisler Selting Werner
(86) Internationale Anmeldenummer: PCT/EP2006/006512
(87) Internationale Veröffentlichungsnummer: WO 2007/003415

(56) Entgegenhaltungen:
- WO-A-03/085086
- WO-A1-03/025580
- SAFAR JIRI G ET AL: "Measuring prions causing bovine spongiform encephalopathy or chronic wasting disease by immunoassays and transgenic mice" NATURE BIOTECHNOLOGY, Bd. 20, Nr. 11, November 2002 (2002-11), Seiten 1147-1150, XP002399820 ISSN: 1087-0156 in der Anmeldung erwähnt
- KIM J I ET AL: "Simple and specific detection of abnormal prion protein by a magnetic bead-based immunoassay coupled with laser-induced fluorescence spectrofluorometry" JOURNAL OF NEUROIMMUNOLOGY, ELSEVIER SCIENCE PUBLISHERS BV, XX, Bd. 158, Nr. 1-2, Januar 2005 (2005-01), Seiten 112-119, XP004672164 ISSN: 0165-5728
- BIESCHKE J ET AL: "ULTRASENSITIVE DETECTION OF PATHOLOGICAL PRION PROTEIN AGGREGATES BY DUAL-COLOR SCANNING FOR INTENSELY FLUORESCENT TARGETS" PROCEEDINGS OF THE NATIONAL ACADEMY OF SCIENCES OF USA, NATIONAL ACADEMY OF SCIENCE, WASHINGTON, DC, US, Bd. 97, Nr. 10, 9. Mai 2000 (2000-05-09), Seiten 5468-5473, XP000999285 ISSN: 0027-8424 in der Anmeldung erwähnt
- PITSCHKE M ET AL: "DETECTION OF SINGLE AMYLOID BETA-PROTEIN AGGREGATES IN THE CEREBROSPINAL FLUID OF ALZHEIMER'S PATIENTS BY FLUORESCENCE CORRELATION SPECTROSCOPY" NATURE MEDICINE, NATURE PUBLISHING GROUP, NEW YORK, NY, US, Bd. 4, Nr. 7, Juli 1998 (1998-07), Seiten 832-834, XP001073409 ISSN: 1078-8956 in der Anmeldung erwähnt
- BERTSCH U ET AL: "Systematic identification of antiprion drugs by high-throughput screening based on scanning for intensely fluorescent targets" JOURNAL OF VIROLOGY, NEW YORK, US, US, Bd. 79, Nr. 12, Juni 2005 (2005-06), Seiten 7785-7791, XP002360668 ISSN: 0022-538X
- BIRKMANN EVA ET AL: "Detection of prion particles in samples of BSE and scrapie by fluorescence correlation spectroscopy without proteinase K digestion" BIOLOGICAL CHEMISTRY, Bd. 387, Nr. 1, Januar 2006 (2006-01), Seiten 95-102, XP002399821 ISSN: 1431-6730

## Beschreibung

Die Erfindung betrifft ein Verfahren zur selektiven Bestimmung der Gegenwart und/ oder der Menge pathologischer Proteinablagerungen des PvP^{sc} in einer Messprobe, die aus einer Körperflüssigkeit aus der Gruppe ausgewählt aus Cerebrospinalflüssigkeit, Lymphflüssigkeit, Blut, Urin und Sputum oder einem Gewebe stammt und die pathologischen Proteinablagerungen des PrP^{SC} mit Scrapie und boviner spongiformer Encephalopathie assoziiert sind.

Eine Reihe von Erkrankungen ist mit dem Auftreten von Proteinablagerungen assoziiert. Bislang ist allerdings weitestgehend unklar, ob diese Proteinablagerungen nur eine Manifestation des jeweiligen Krankheitsbildes darstellen oder in der Tat kausal für die Ätiologie und/oder Progression dieser Erkrankungen verantwortlich sind. So sind beispielsweise neurodegenerativen Erkrankungen bekannt, bei denen im Gehirn der Betroffenen als amyloide Plaques bezeichnete Proteinablagerungen auftreten. Zu diesen Erkrankungen zählen unter anderem Morbus Alzheimer, Morbus Parkinson, Chorea Huntington, vererbbare cerebrale amyloide Angiopathie sowie die transmissiblen spongiformen Encephalopathien. Zur letzteren zählen zum Beispiel die Creutzfeldt-Jakob-Erkrankung (CJD) beim Menschen, Scrapie bei Schafen oder die bovine spongiforme Enzephalopathie (BSE) bei Rindern sowie weitere, früher als "Slow Virus"-Erkrankungen bezeichnete Syndrome, wie die Kuru-Erkrankung. Heute werden diese Krankheiten unter dem Begriff Prionenerkrankungen zusammengefasst (Übersichten in Prusiner, S.B. (1982) Science 216, 136-144; Weissmann, C. (1996) FEBS Lett. 389, 3-11; Riesner, D. (1996) Chemie in unserer Zeit, S. 66-74; Prusiner, S.B. (1998) Proc. Natl. Acad. Sci. USA 95, 13363-13383).

Pathologische Proteinablagerungen treten jedoch nicht nur bei Erkrankungen des neuronalen Systems in Erscheinung, sondern werden auch in anderen Organen beobachtet. Beispielsweise wird bei Typ II Diabetes mellitus bei einigen Patienten eine diabetische Nephropathie beobachtet, als deren Grund eine Matrixstörung durch Proteinablagerungen diskutiert wird. Eine Übersicht nicht neuronaler Erkrankungen, die mit der Bildung von pathologischen Proteinablagerungen einhergehen, findet sich in Sipe, W. (1992) Annu. Rev. Biochem. 61, 947-975.

Bei den transmissiblen spongiformen Encephalopathien werden Ablagerungen der infektiösen Form des Prionproteins (PrP^{Sc}) kausal mit der Krankheitsentstehung in Verbindung gebracht. Dieses modifizierte Prionprotein ist in der Lage, mit der normalen zellulären Form PrP derart in Wechselwirkung zu treten, dass die infektiöse Form PrP^{Sc} eine Konformationsänderung der Wildtyp-Form PrP zur infektiösen Form bedingt. Die infektiöse Formen PrP^{Sc} aggregieren und bilden die für die Indikation charakteristischen pathologischen Proteinablagerungen aus.

In den letzten Jahren ist vor allem die bovine spongiforme Encephalopathie (BSE) ins Bewusstsein der Öffentlichkeit gerückt, insbesondere auch deshalb weil BSE mit der humanen Creutzfeldt-Jakob-Erkrankung in Verbindung gebracht wird. Der Etablierung von Nachweisverfahren zur Diagnose von Prionenerkrankungen kommt daher besondere Bedeutung zu. Zum Beispiel besteht aus veterinärmedizinischer Sicht die Notwendigkeit sicherzustellen, dass keine kontaminierten Produkte, beispielsweise Fleisch, von mit BSE infizierten Rindern oder mit Scrapie infizierten Schafen in Umlauf gerät. Wünschenswert ist neben einer sicheren und schnellen Diagnose eine hohe Sensitivität des Nachweises, sodass infizierte Tiere frühzeitig erkannt werden können. Weiterhin sollte die Vorbereitung des biologischen Probenmaterials einen möglichst geringen Arbeitsaufwand erfordern, um dadurch auch eine effiziente Durchführung von Reihenuntersuchungen zu ermöglichen. Für eine sichere Früherkennung von Prionenerkrankungen ergibt sich weiterhin die Notwendigkeit, Verluste an pathogenem Material bei der Vorbereitung des Probenmaterials zu vermeiden. Gängige BSE-Testverfahren (siehe u.a. Hörnlimann, B. et al. (2001) Prionen und Prionkrankheiten, Gruyter, 290-295; Pitschke, M. et al. (1998) Nat. Med. 4, 832-834; Safar, J.G. et al. (1998) Nat. Med. 4, 1157-1165; Bieschke, J. et al. (2000) Proc. Natl. Acad. Sci. USA 97, 5468-5473; Safar, J.G. et al. (2002) Nat. Biotechnol. 20, 1147-1150; Thomzig, A. et al. (2004) J. Biol. Chem. 279, 33847-33854) nutzen als wesentliches Kriterium die Proteinase K (PK)-Resistenz von infektiösem PrP^{Sc}. Der Anteil an PK-resistentem PrP^{Sc} schwankt allerdings sowohl zwischen Individuen als auch zwischen verschiedenen Arealen eines Organs desselben Individuums mitunter sehr stark. Zudem ist der Anteil von PK-resistentem PrP^{Sc} in BSE-infizierten Rindern niedriger als in Scrapie-infizierten Schafen. Das bedeutet aber, dass eine Behandlung des Probenmaterials mit Proteinase K zu einer nicht unerheblichen Verzerrung der Messergebnisse führen kann, da Teile des pathogenen Materials nicht erfasst werden. Daher ist es für ein sensitives Nachweisverfahren wünschenswert, die infektiösen Prionproteine bzw. deren gegenseitige Assoziation direkt zu messen.

Es ist ferner unbekannt, wie das Verhältnis von PKsensitivem zu PK-resistentem Prp^{Sc} in frühen Stadien der Krankheit ist.

Eine weitere Herausforderung im Zusammenhang mit der Infektionskontrolle von Prionerkrankungen liegt darin, dass zumindest hypothetisch das Risiko besteht, dass zum Beispiel BSE-infiziertes Material über kontaminiertes Futter (u.a. Fleisch, Knochen- oder Tiermehl) in andere Spezies, etwa Schafe, übertragen werden kann. Die klinischen Symptome von Scrapie können jedoch nicht mit hinreichender Genauigkeit von denen experimentell in Schafen erzeugter BSE unterschieden werden (Bradley, R. (2004) Prions: A Challenge for Science, Medicine, and the Public Health System. (Rabenau, H.F., Cinatl, J., and Doerr, H.W., Hrsg.) S. Karger AG, Basel, Schweiz, S. 146-185). Bislang ist dazu eine zeitaufwendige und kostenintensive Typisierung in Mausmodellen notwendig. Daher besteht ein Bedarf an Verfahren, mit denen selektiv ein Prionprotein einer bestimmten Spezies nachgewiesen werden kann.

Die entsprechenden Anforderungen an ein Nachweisverfahren für pathologische Proteinablagerungen sind nicht auf den Nachweis von Prionen beschränkt und auf den Nachweis weiterer Erkrankungen übertragbar, die mit Proteinablagerungen in Verbindung stehen.

Das Europäische Patent EP1015888 offenbart ein Verfahren, das die Assoziation von Teilstrukturen der Proteinablagerungen als Target (Zielstruktur) an eine Sonde, die zur Assoziation mit dem Target befähigt ist, direkt misst. Unter einer Teilstruktur ist dabei ein pathologisches Protein per se oder eine Aggregation mehrerer Proteine zu verstehen, welche pathologische Proteine beinhaltet. Die Wechselwirkung einer solchen Teilstruktur an eine Sonde wird bevorzugt spektroskopisch erfasst, wobei als Sonde verschiedene Strukturen eingesetzt werden können, die zur Wechselwirkung mit der Teilstruktur befähigt sind. Folglich kann daher aufgrund der Selbstaggregation von Prionproteinen in diesem Fall auch das pathologische Protein selbst als Sonde dienen. Die Proteinaggregate können auf Grund intrinsischer Eigenschaften der betreffenden Moleküle direkt detektiert werden oder werden durch Anlagerung von beispielsweise fluoreszenzmarkierten Antikörpern und/oder fluoreszierenden, synthetisch hergestellten Sondenmolekülen und Anregung dieser fluoreszierenden Verbindungen durch Laserlicht indirekt nachgewiesen.

Das in EP1015888 offenbarte Verfahren weist jedoch analog zu den oben zitierten anderen gängigen Nachweisverfahren den Nachteil auf, dass die markierten Targets (Zielstrukturen), also etwa infektiöse Prionenaggregate, frei beweglich in Lösung gemessen werden. Die Aggregate liegen dabei in aller Regel in sehr geringer Konzentration und nicht gleich verteilt in der Messprobe vor. Größere Aggregate sinken zum Beispiel schneller ab und verschwinden so aus dem messbaren Volumen. Dieser Sachverhalt bedingt somit zum Teil erhebliche Messungenauigkeiten.

Der vorliegenden Erfindung liegt daher das Problem zugrunde, ein verbessertes Verfahren zum Nachweis von pathologischen Proteinablagerungen bereitzustellen, das im Vergleich mit den bekannten Nachweisverfahren nicht nur eine höhere Messgenauigkeit in Verbindung mit einer gesteigerten Sensitivität aufweist, sondern auch die selektive Bestimmung einer spezifischen Proteinablagerung erlaubt.

Dieses Ziel wird durch ein Verfahren zur selektiven Bestimmung der Gegenwart und/oder der Menge pathologischer Proteinablagerungen gemäß dem unabhängigen Anspruch 1 erreicht, das umfasst:
(a) Immobilisieren eines Fängermoleküls, das spezifische Bindungsaffinität für Teilstrukturen der zu bestimmenden Proteinablagerungen aufweist, auf einer Oberfläche;
(b) In-Kontakt-bringen des immobilisierten Fängermoleküls mit einer Messprobe, von der vermutet wird, dass sie pathologische Proteinablagerungen oder Teilstrukturen davon enthält;
(c) Inkubieren des Ansatzes, sodass die Bildung eines Komplexes aus dem immobilisierten Fängermolekül und den Teilstrukturen der zu bestimmenden Proteinablagerungen ermöglicht wird;
(d) In-Kontakt-bringen des Komplexes mit mindestens einer detektierbaren Einheit, die spezifische Bindungsaffinität für die Teilstrukturen der zu bestimmenden Proteinablagerungen aufweist, und die ein optisch detektierbares Signal erzeugt, wobei mindestens eine der mindestens einen detektierbaren Einheit ein mittels spektroskopischer Verfahren detektierbares Signal erzeugt; und
(e) Detektieren der Komplexbildung durch Messung des von der mindestens einen detektierbaren Einheit insgesamt erzeugten Signals.

Die Erfindung basiert dabei auf der überraschenden Entdeckung, dass durch eine Immobilisierung der zu bestimmenden Proteinablagerung auf einer Oberfläche in Kombination mit der Verwendung spezieller Fängermoleküle und Detektionseinheiten ein selektives und effizientes Nachweisverfahren für Proteinablagerungen mit hoher Sensitivität etabliert werden konnte, das sämtliche oben erwähnte Nachteile der bislang bekannten Verfahren überwindet.

Das erfindungsgemäße Verfahren ist gekennzeichnet durch die Immobilisierung der zu bestimmenden Proteinablagerungen auf einer Oberfläche. Dadurch erfolgt eine Konzentration der Proteinaggregate in der Fläche, die eine erhebliche Steigerung der Testsensitivität bedingt. Gleichzeitig ist es möglich, die gesamte Fläche abzurastern (zu scannen) und somit sämtliche immobilisierten Proteinaggregate zu detektieren, insbesondere auch einzeln, und zu zählen, während bei einer dreidimensionalen Messung in Lösung häufig nur ein Teilvolumen analysiert und somit ein erheblicher Anteil der vorhandenen Proteinaggregate nicht erfasst wird. Zudem wird ein Absinken von größeren Proteinaggregaten aus dem Messbereich verhindert. Beide Aspekte bedingen eine signifikante Verbesserung der Messgenauigkeit.

Die Proteinablagerungen können auf einer beliebigen Oberfläche immobilisiert werden, beispielsweise einer Glasoberfläche, einer Kunststoffoberfläche oder einer Metalloberfläche. Vorzugsweise werden die Proteinaggregate auf einem Analyse- oder Assay-Chip immobilisiert. Derartige Chips sind von zahlreichen Anbietern kommerziell verfügbar.

Die Immobilisierung an die Oberfläche erfolgt über ein Fängermoleküle ("Capture-Molekül"), das spezifische Bindungsaffinität für Teilstrukturen der zu bestimmenden Proteinablagerungen aufweist, d.h. das diese Teilstrukturen auch im Vergleich zu ähnlichen oder homologen Teilstrukturen mit deutlich besserer Affinität bindet. In anderen Worten, das Fängermolekül unterscheidet ähnliche Strukturen, wodurch die Spezifität bzw. Selektivität des Nachweises weiter erhöht wird.

Die Fängermoleküle sind kovalent oder nicht-kovalent an die Oberfläche gebunden. In bestimmten Ausführungsformen der Erfindung wird die Oberfläche vor der Immobilisierung der Proteinaggregate aktiviert. Eine solche Aktivierung kann zum Beispiel durch Abflammen und durch Beschichten der Oberfläche mit verschiedenen Polymeren, zum Beispiel mit poly-D-Lysin, erreicht werden.

Unter Teilstrukturen der zu bestimmenden Proteinablagerungen, für welche die Fängermoleküle spezifische Bindungsaktivität besitzen, werden monomere oder oligomere Einheiten der Proteinablagerungen verstanden, zum Beispiel monomere Prionproteine oder oligomere Proteinaggregate. Bei einer erfindungsgemäßen Teilstruktur kann es sich aber auch um einen Teil eines Monomers, z.B. ein Peptid, handeln.

Bevorzugt werden Fängermoleküle, die aus der Gruppe ausgewählt werden, welche aus monoklonalen Antikörpern, polyklonalen Antikörpern und Antikörperfragmenten besteht, wobei monoklonale Antikörper besonders bevorzugt sind.

Durch Verwendung spezifischer monoklonaler Antikörper können zum Beispiel Scrapie- bzw. BSE-spezifische Prionproteine in derselben Messprobe jeweils selektiv an einer Oberfläche immobilisiert und nachgewiesen werden.

In anderen Ausführungsformen besteht das Fängermolekül selbst ebenfalls aus Teilstrukturen pathologischer Proteinablagerungen oder aus Fragmenten davon, wobei diese Teilstrukturen natürlichen Ursprungs oder rekombinant hergestellt sein können. Aufgrund der Selbstaggregation, etwa von Prionproteinen, werden so die zu detektierenden Proteinablagerungen ebenfalls an der Oberfläche immobilisiert. Dabei können als Fängermolekül sowohl Teilstrukturen der zu bestimmenden Proteinablagerung verwendet werden (homologes System) als auch Teilstrukturen anderer Proteinablagerungen (heterologes System). Beispielsweise kann eine Teilstruktur, die aus amyloiden Plaques einer BSE stammt, als Fängermolekül zur Detektion von Teilstrukturen aus an Alzheimer erkranktem Gewebe verwendet werden.

Optional können nach Immobilisierung des Fängermoleküls noch verbliebene freie Bindungsstellen auf der Oberfläche durch Inkubation mit einem Blockierungsreagenz blockiert werden, um die unspezifische Bindung der zu bestimmenden Proteinaggregate zu reduzieren. Als Blockierungsreagenzien können zum Beispiel Rinderserumalbumin (BSA)-Lösung oder fettfreie Magermilch verwendet werde.

Gemäss der Erfindung sind die zu detektierenden Proteinablagerungen des PvP^{Sc} mit Scrapie und boviner spongiformer Encephalopathie assoziiert

Die zu bestimmenden PvP^{sc} Proteinablagerungen können in einer beliebigen biologischen Messprobe nachgewiesen werden, die zum Beispiel aus einer Körperflüssigkeit oder einem Gewebe stammt. In bestimmten Ausführungsformen der Erfindung wird die Körperflüssigkeit aus der Gruppe ausgewählt wird, die aus Cerebrospinalflüssigkeit, Lymphflussigkeit, Blut, Urin und Sputum besteht, wobei Cerebrospinalflüssigkeit und Blut besonders bevorzugt werden. In anderen bevorzugten Ausführungsformen stammt die Messprobe aus Hirngewebe.

In anderen bevorzugten Ausführungsformen wird die Messprobe vor dem In-Kontakt-bringen mit dem Fängermolekül einem Reinigungsverfahren unterworfen, um die zu bestimmenden Proteinablagerungen von etwaig vorhandenen Kontaminanten zu isolieren. In Abhängigkeit von der Art der Probe, der vermuteten Konzentration der zu bestimmenden Proteinablagerungen, der verwendeten Detektionsmethode und dergleichen kann eine partielle oder eine (nahezu) vollständige Reinigung/Isolation durchgeführt werden. Die Proben können dabei mit physikalischen und/oder chemischen Standardmethoden (z.B. Ultraschall, Temperaturänderungen, Inkubation mit Lösungen unterschiedlicher Ionenstärke, chaotropen Salzen Detergenzien und Enzymen) behandelt werden. Dabei können die Methoden einzeln oder in beliebiger Kombination angewendet werden.

In bevorzugten Ausführungsformen der Erfindung werden die Messproben durch Natriumphosphorwolframat (NaPTA)-Fällung gereinigt (Safar, J.D. et al. (1998) Nat. Med. 4, 1157-1165), wobei auf eine Zugabe von Proteasen, insbesondere von Proteinase K, verzichtet wird.

Die Messprobe wird anschließend erfindungsgemäß mit dem immobilisierten Fängermolekül inkubiert, um die Bildung eines Komplexes aus dem immobilisierten Fängermolekül und den Teilstrukturen der zu bestimmenden Proteinablagerungen zu ermöglichen.

Der Nachweis der Komplexbildung zwischen Fängermolekül und zu detektierenden Teilstruktur erfolgt durch In-Kontakt-bringen des Komplexes mit zwei detektierbaren Einheiten. Unter einer detektierbaren Einheit ist ein Sondenmolekül zu verstehen, das eine spezifische Bindungsaffinität für die Teilstrukturen der zu bestimmenden Proteinablagerungen aufweist, und die ein optisch detektierbares Signal erzeugt, wobei das optisch detektierbare Signal durch das Sondenmolekül selbst erzeugt werden kann oder durch einen Bindungspartner, der an das Sondenmolekül gekoppelt ist. Beispiele für solche Bindungspartner sind radioaktive fluoreszierende, chemilumineszente oder biolumineszente Markierungen sowie Metallpartikel (z.B. kolloidales Gold). Die zwei detektierbaren Einheiten erzeugten ein mittels spektroskopischer Verfahren detektierbares Signal.

Gemäss der Erfindung werden zwei detektierbare Einheiten simultan mit dem Komplex in Kontakt gebracht. Dabei kann das In-Kontakt-bringen mit dem Komplex simultan oder seriell erfolgen.

In bevorzugten Ausführungsformen der Erfindung umfassen die detektierbaren Einheiten ein. Protein oder ein Polypeptid, wobei diese Protein oder Polypeptid natürlichen Ursprungs oder rekombinant hergestellt sein kann. In besonders bevorzugten Ausführungsformen wird das Protein oder Polypeptid aus der Gruppe ausgewählt, die aus monoklonalen Antikörpern, polyklonalen Antikörpern und Antikörperfragmenten besteht, wobei monoklonale Antikörper bevorzugt sind. Die detektierbaren Einheiten können jedoch zum Beispiel auch kleine organische Moleküle, Nukleinsäuren (einzel- oder doppelsträngige DNA oder RNA) oder Polysaccharide umfassen.

Gemäss der Erfindung wird das von den detektierbaren Einheiten erzeugte optisch detektierbare Signal aus der Gruppe ausgewählt, die aus Fluoreszenz-, Chemilumineszenz- und Biolumineszenzemission besteht.

In besonders bevorzugten Ausführungsformen bestehen die detektierbaren Einheiten aus fluoreszenzmarkierten Antikörpern, insbesondere monoklonalen Antikörpern.

Optional können nach dem In-Kontakt-bringen der detektierbaren Einheiten mit dem Komplex ein oder mehr Waschschritte durchgeführt werden, um nicht gebundene detektierbare Einheiten zu entfernen, welche ansonsten mit dem Messergebnis interferieren können.

Die Komplexbildung wird durch Messung der von den mindestens zwei detektierbaren Einheiten insgesamt erzeugten Signale detektiert. Der Begriff "insgesamt erzeugte Signale" bedeutet dabei, dass bei Verwendung mehr als einer detektierbarer Einheit die optisch detektierbaren erzeugten Einzelsignale erfasst (Koinzidenzmessung), miteinander korreliert und ausgewertet werden. Dadurch wird die Spezifität des Nachweises erhöht, da positive Signale nur dann auftreten, wenn die verschiedenen detektierbaren Einheiten gleichzeitig an den nachzuweisenden Komplex gebunden sind.

Erfindungsgemäβ werden die zu bestimmenden PvP^{Sc} Proteinablagerungen mit Hilfe von fluoreszenzkorrelationsspektroskopie (FCS) in Kombination mit Kreuzkorrelation und Single Particle Immunosorband Laserscanning Assay detektiert.

Hierbei regt ein Laserstrahl die nachzuweisenden Proteinkomplexe zu starkem Fluoreszenzlicht an, welches mit Hilfe eines Detektors, bevorzugt einer konfokalen Optik, registriert wird. Auf Grund der optischen Eigenschaften des Systems wird ein Einzemolekülnachweis ermöglicht, sodass individuelle Proteinaggregate gezählt werden können. Wie bereits erwähnt, wird über eine Kreuzkorrelation die Sensitivität und Spezifität des Nachweises zusätzlich gesteigert, indem beispielsweise zwei verschiedene monoklonale Antikörper, die mit unterschiedlichen Fluoreszenzfarbstoffen markiert sind, benutzt werden und die gleichzeitige Bindung über Kreuzkorrelation der Signale bestimmt wird.

Diese spektrokopischen Detektionsverfahren werden jeweils in Verbindung mit entsprechenden Auswerteverfahren, z.B. Fluoreszenzintensitätsdistributionsanalyse, angewendet.

Durch die Immobilisierung der zu bestimmenden Proteinablagerungen auf einer Oberfläche ist es im Gegensatz zu einer Bestimmung in Lösung möglich, das von der mindestens einen detektierbaren Einheit erzeugte Gesamtsignal durch Abrastern ("Scannen") der Oberfläche zu messen, zum Beispiel mittels eines Single Particle Immunosorband Laserscanning Assays, wobei eine Mehrzahl nebeneinander liegender Teilbereiche der Oberfläche abgerastert wird, und die Einzelwerte anschließend addiert werden

Die Erfindung wird ferner durch die folgenden nicht einschränkenden Figuren und Beispiele veranschaulicht.

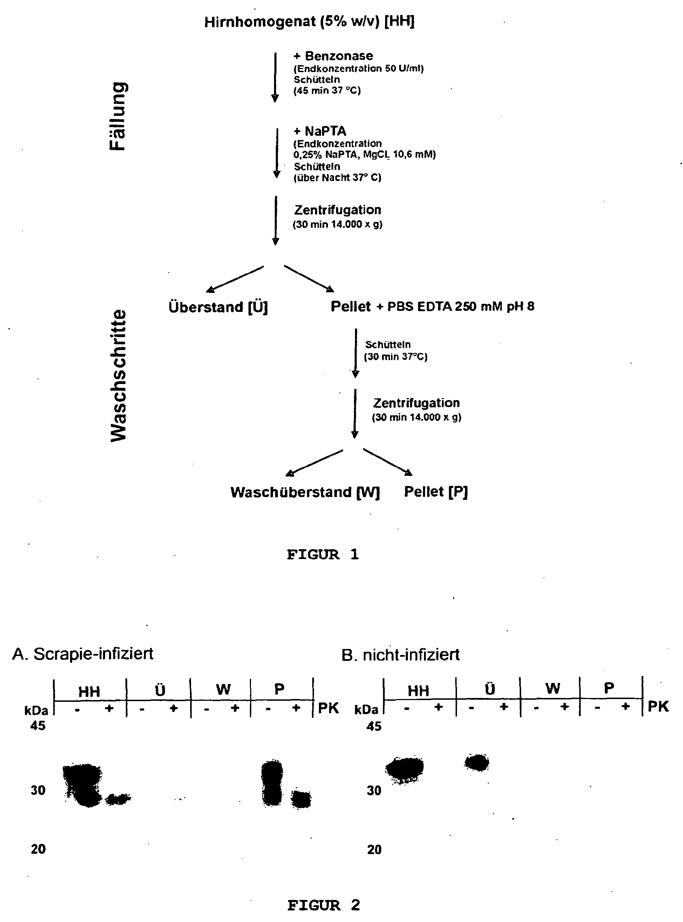

- **Fig. 1**: zeigt eine schematische Darstellung des für die Reinigung von PrP^{Sc} aus Hamsterhirngewebe mittels NaPTA-Fällung ohne Proteinase K-Zugabe verwendeten Protokolls.
- **Fig. 2**: zeigt eine Western Blot-Analyse der einzelnen Schritte der NaPTA-Fällung zur Proteinase K-freien Reinigung von pathogenem PrP aus Scrapie-infiziertem Hamsterhirn Die Western Blot-Analyse nach SDS-PAGE zeigt jeweils gleiche Mengen (1 x 10⁻³ gÄ) der einzelnen Reinigungsschritte der NaPTA-Fällung. **HH:** Hirnhomogenat, **Ü:** Überstand, **W:** Überstand aus Waschschritt, **P**: resultierendes Pellet. Von jedem Schritt sind Kontrollen, welche proteolytisch für eine Stunde bei 37°C mit 5 µg/ml Proteinase K (PK) behandelt wurden (**+**) aufgetragen. Die Reinigung wurde mit Scrapie-infiziertem Hirnhomogenat (**A**) und mit einer nicht infizierten Hirnprobe (**B**) dargestellt

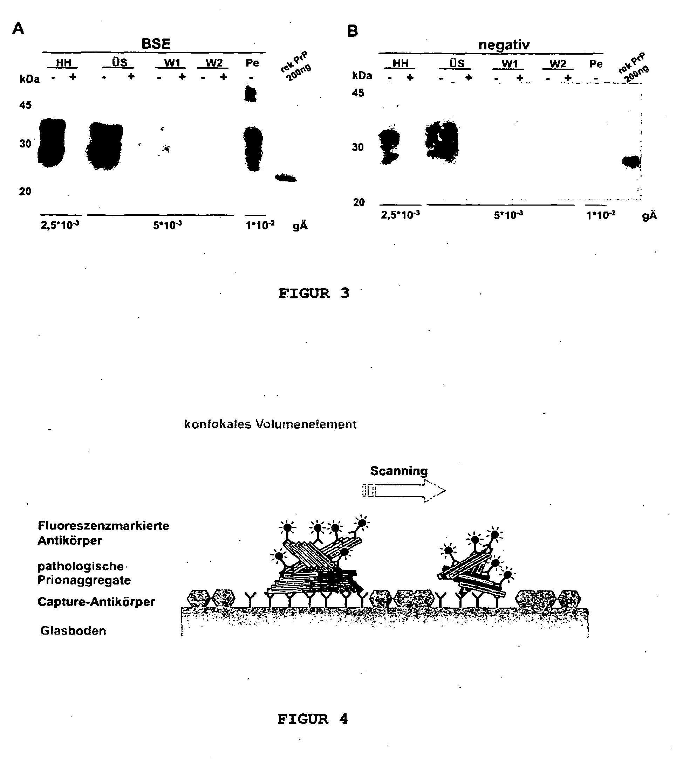

- **Fig. 3**: zeigt eine Western Blot-Analyse der NaPTA-Fällung zur Reinigung von pathogenem PrP^{BSE} aus der *medulla oblongata* eines BSE-infizierten Rindes. Die Western Blot-Analyse nach SDS-PAGE zeigt die jeweiligen Mengen (2.5 x 10⁻³ - 1 x 10⁻² gÄ) der einzelnen Reinigungsschritte der NaPTA-Fällung. Die Reinigung wurde mit Proben einer BSE-infizierten *medulla oblongata* vom Rind **(A)** und einer nicht infizierten Kontrolle **(B)** durchgeführt. **HH:** Hirnhomogenat, **ÜS:** Überstand, **W1-2:** Überstand aus Waschschritt, **P:** resultierendes Pellet. Von jedem Schritt sind jeweils Kontrollen, welche proteolytisch für eine Stunde bei 37°C mit 5 µg/ml PK behandelt wurden (**+**) aufgetragen. Als Kontrolle ist in beiden Fällen 200 ng bovines rekombinantes PrP(29-231) aufgetragen (rekPrP).
- **Fig. 4**: zeigt eine schematische Darstellung eines Single Particle Immuno-sorband Laserscanning Assays (SPILA).

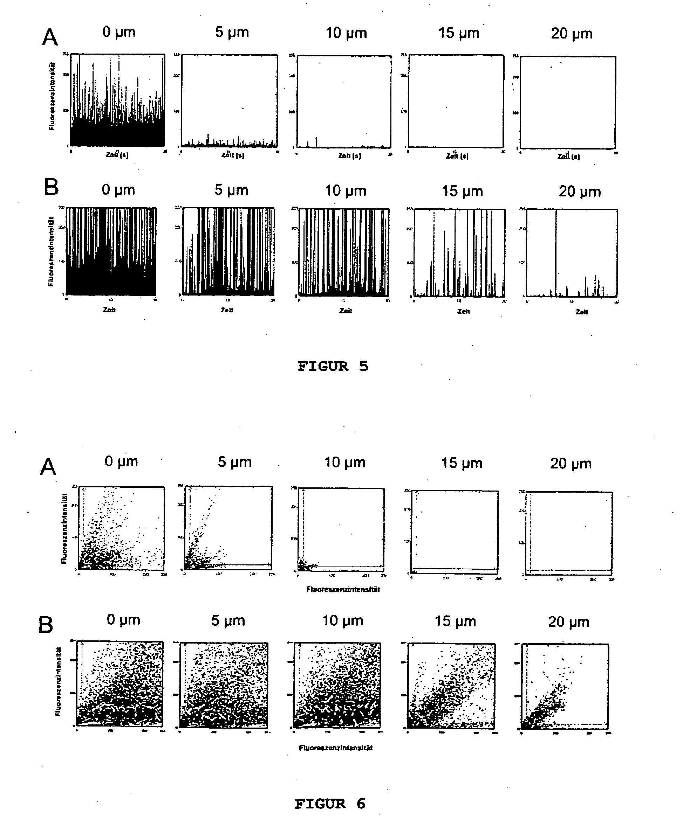

- **Fig. 5**: zeigt Fluoreszenzintensitätsmessungen in Abhängigkeit von der Höhe des Fokus der FCS-Optik über der Oberfläche ("Höhenscans") immobilisierter negSHa- (**A**) bzw. PrP^{Sc}-Proben (**B**). Als Sonde zur Detektion wurde fluoreszenzmarkierter (Alexa 633) D13 Antikörper (Inpro, USA) eingesetzt. Die Proben wurden im Abstand von 0-20 µm von der Chipoberfläche in 5 µm-Schritten für jeweils 30 Sekunden im FCS gemessen.
- **Fig. 6**: zeigt 2D-FIDA Plots von Fluoreszenzintensitätsmessungen immobilisierter negSHa- (**A**) bzw. PrP^{Sc}-Proben (**B**). Als Sonden zur Detektion wurden die fluoreszenzmarkierten Antikörper R1 und D13 (Alexa 488-markierter R1, Alexa 633-markierter D13; Inpro, USA) eingesetzt. Die Proben wurden im Abstand von 0-20 µm von der Chipoberfläche in 5 µm-Schritten für jeweils 30 Sekunden im FCS gemessen.

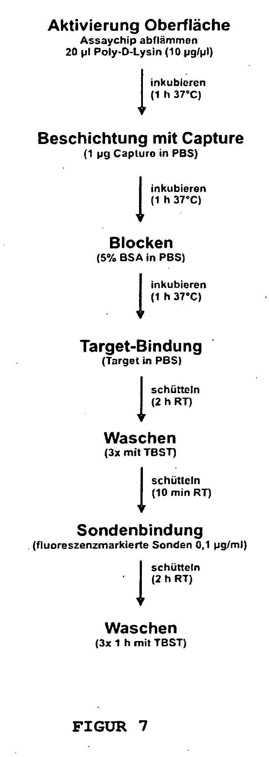

- **Fig. 7**: zeigt schematisch die Schritte des Protokolls zur Immobilisierung und Markierung pathologischer Prionenaggregate.

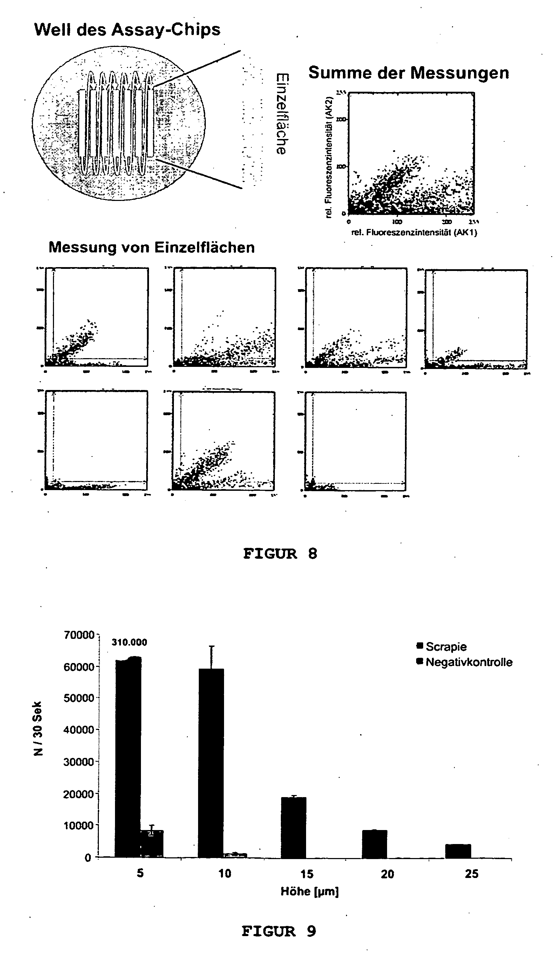

- **Fig. 8**: zeigt die Bestimmung und Summierung von sieben definierten Flächen einer immobilisierten PrP^{Sc}-Probe. Die Messung wurde im Abstand von 15 µm über der Chipoberfläche durchgeführt. Als Sonden zur Detektion wurden die fluoreszenzmarkierten Antikörper R1 und D13 (Alexa 488-markierter R1, Alexa 633-markierter D13; Inpro, USA) eingesetzt. Die Messungen wurden für jeweils 30 Sekunden an sieben nebeneinander liegenden Flächen unter "Scanningbewegung" (Abrastern) durchgeführt. Die Ergebnisse der Einzelmessungen wurden summiert.
- **Fig. 9**: zeigt die Auswertung von 2D-FIDA Messungen durch eine immobilisierte PrP^{Sc}-Probe (**rot**) sowie eine Negativkontrolle (**grün**). Als Fängermolekül wurde der Antikörper R1, als Detektionssonden die fluoreszenzmarkierten Antikörper R1 und D13 (Alexa 488-markierter R1, Alexa 633-markierter D13; Inpro, USA) eingesetzt. Die Proben wurden im Abstand von 5-25 µm von der Chipoberfläche in 5 µm-Schritten für jeweils 30 Sekunden im FCS gemessen.

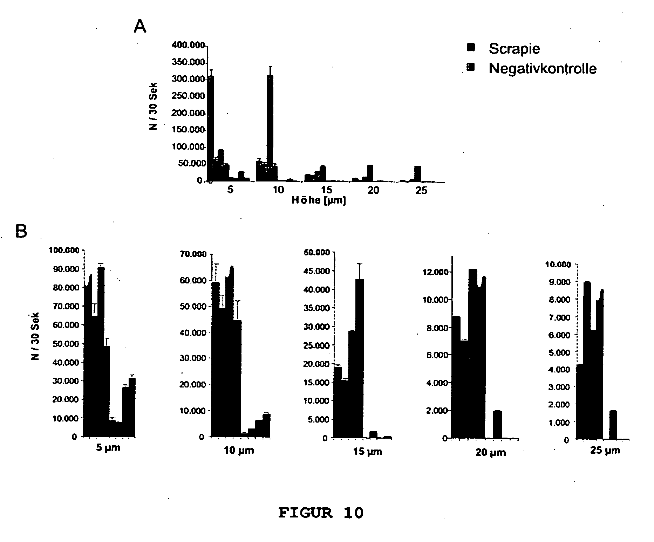

- **Fig. 10**: zeigt eine Messung ("Höhenscan") von jeweils vier immobilisierten PrP^{Sc}-Proben (**rot**) bzw. Negativkontrollen (**grün**) via 2D-FIDA Messung. Als Fängermolekül wurde der Antikörper R1 und als Sonden zur Detektion die fluoreszenzmarkierten Antikörper R1 und D13 (Alexa 488-markierter R1, Alexa 633-markierter D13; Inpro, USA) eingesetzt. Die Proben wurden im Abstandsbereich von 5-25 µm von der Chipoberfläche in 5 µm-Schritten für jeweils 30 Sekunden im FCS gemessen. (**A**) Gesamtheit aller Messungen in den unterschiedlichen Distanzen zur Chipoberfläche, (**B**) Darstellung der Messungen nach Höhen 5-25 µm getrennt mit einer vergrößerten Ordinate, wobei die jeweils größten Werte abgeschnitten sind.

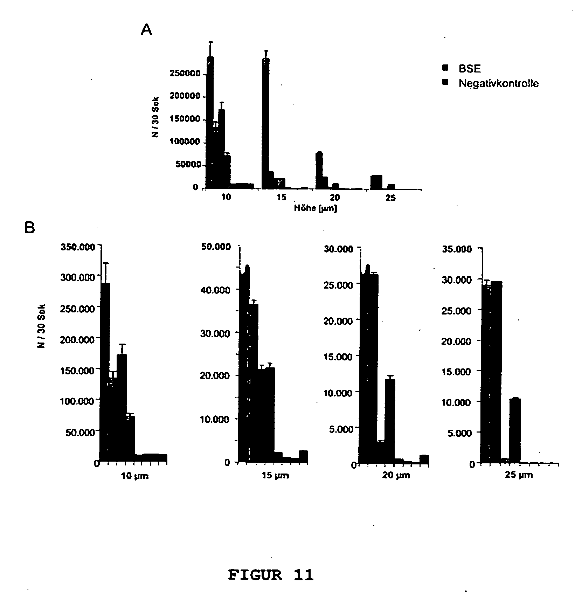

- **Fig. 11**: zeigt eine Messung ("Höhenscan") von jeweils vier immobilisierten PrP^{BSE}-Proben (**rot**) bzw. Negativkontrollen (**grün**) via 2D-FIDA Messung. Als Fängermolekül wurde der Antikörper Saf32 und als Detektionssonden die fluoreszenzmarkierten Antikörper 12F10 und Saf32 (Alexa 488-markierter 12F10, Alexa 633-markierter Saf32; Spibio, USA) eingesetzt. Die Proben wurden im Abstand von 10-25 µm von der Chipoberfläche in 5 µm-Schritten für jeweils 30 Sekunden im FCS gemessen. (**A**) Gesamtheit aller Messungen in den unterschiedlichen Distanzen zur Chipoberfläche, (**B**) Darstellung der Messungen nach Höhen 10-25 µm getrennt mit einer vergrößerten Ordinate, wobei die jeweils größten Werte abgeschnitten sind.

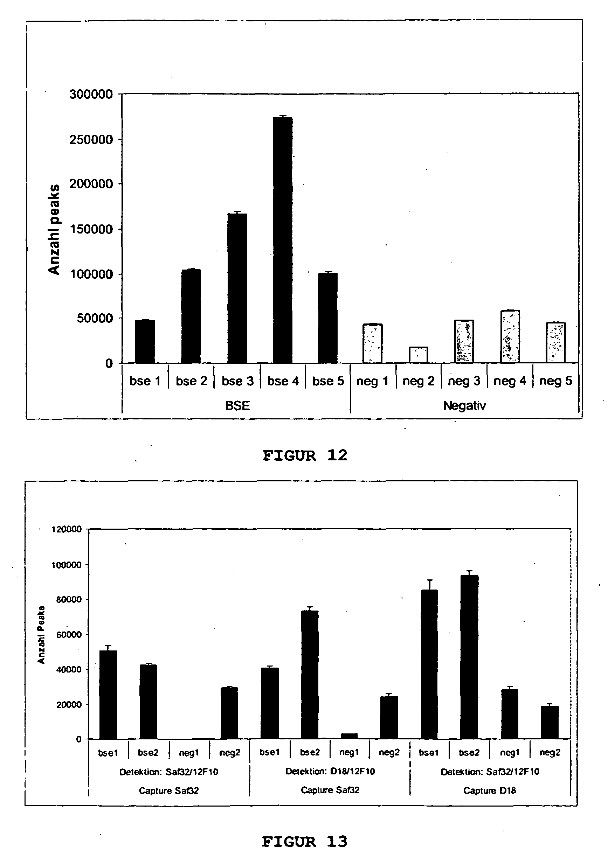

- **Fig. 12**: zeigt eine 2D-FIDA-Messung immobilisierter BSE-Liqourproben und Negativkontrollen. Der Immobilisierungsansatz wurde mit fünf PrP^{BSE}-Liqour Proben (bse 1-5) sowie fünf Negativkontrollen (neg 1-5) durchgeführt. Als Fängermolekül wurde der Antikörper Saf32 und als Detektionsonden die fluoreszenzmarkierten Antikörper 12F10 und Saf32 eingesetzt. Die Proben wurden 5 µm über der Glasbodenoberfläche gemessen.
- **Fig. 13**: zeigt eine 2D-FIDA-Messung immobilisierter BSE-Liqourproben und Negativkontrollen. Der Immobilisierungsansatz wurde mit zwei PrP^{BSE}-Liqourproben (bse 1 und bse 2) sowie zwei Negativkontrollen (neg 1 und neg 2) durchgeführt. Als Fängermolekül wurden die Antikörper Saf32 sowie D18 und als Detektionsonden die fluoreszenzmarkierten Antikörper 12F10, Saf32 und D18 in den jeweils angegebnen Kombinationen eingesetzt. Die Proben wurden 5 µm über der Glasbodenoberfläche gemessen

### Beispiel 1: Reinigung von PrP^{Sc} aus Hamsterhirngewebe ohne Proteolyse

Die Aufreinigung von PrP^{Sc} (Fig. 1) erfolgte in Anlehnung an ein Protokoll von Safar und Kollegen (Safar, J. et al. (1998) Nat. Med. 4, 1157-1165). Hirngewebe von Scrapie-infizierten syrischen Goldhamstern sowie von nicht infizierten Kontrollproben wurde von der RKI Berlin (Dr. M. Beekes) und der UCSF, San Francisco, USA (Dr. S. Prusiner) erhalten. Das Hirngewebe in PBS mit 2 % Sarkosyl wurde mit Hilfe eines Homogenisators (PowerGen 125, Fisher Scientific) zu einem 5 % (w/v) Homogenat verarbeitet. Dieses wurde für eine Minute bei 5.000 x g zentrifugiert, um größere Gewebefragmente zu sedimentieren. Anschließend wurde Benzonase (Merck, Darmstadt) bis zu einer Endkonzentration von 50 U/ml zugesetzt, um DNA und RNA abzubauen (Inkubation unter Schütteln für 45 Minuten bei 37°C). Danach wurden NaPTA (Endkonzentration 0.25 %) und MgCl₂ (Endkonzentration 10.6 mM) zugegeben, und der Fällungsansatz wurde über Nacht bei 37° C geschüttelt. Anschließend wurde die Probe für 30 Minuten bei 14.000 x g zentrifugiert und der Überstand verworfen. Das Pellet wurde mit 250 µl PBS/250 mM EDTA für mindestens 30 Minuten bei 37 °C gewaschen und erneut für 30 Minuten bei 14.000 x g zentrifugiert. Dieser Waschschritt wurde zweimal wiederholt. Die Verwendung von PBS/250 mM EDTA, pH 8 als Waschpuffer erbrachte im Vergleich zu bekannten Waschpuffern mit nur 50 mM EDTA, die von Wadsworth und Kollegen (Wadsworth, J.D. et al. (2001) *Lancet* **358**, 171-180) für die PrP^{CJD}-Reinigung aus menschlichem Hirngewebe von CJD-Patienten beschrieben wurden, eine erheblich verbesserte Aufreinigung.

Eine Western Blot-Analyse der Reinigungsschritte der NaPTA-Fällung von Scrapie-infizierten und nicht infizierten Hirnproben ist in Fig. 2 dargestellt. In der Pelletfraktion der Aufreinigung aus Hirngewebe des Scrapie-infizierten Hamsters war eine große PrP-Menge nachweisbar. Ein Vergleich mit der Pelletfraktion der Probe des nicht infizierten Hamsters, die kein PrP enthielt, lässt den Schluss zu, dass es sich bei dem PrP der Scrapie-infizierten Probe ausschließlich um pathogenes PrP handelt. Darauf deutet auch die Proteinase K-Resistenz des größten Teils des gefällten PrPs hin. Fig. 2B zeigt, dass PrP^{c} fast vollständig im Überstand verbleibt.

Damit konnte gezeigt werden, dass durch die Fällung große Teile des vorhandenen pathogenen PrPs im Gegensatz zum natürlichen PrP^{c} selektiv angereichert und konzentriert werden konnten. Durch die einzelnen Waschschritte wurde kein Verlust an pathogenem PrP hervorgerufen.

### Beispiel 2: Reinigung von PrP^{BSE} aus der medulla oblongata des Rinderhirns ohne Proteolyse

BSE-infizierte Gewebeproben aus der *medulla oblongata* des Rindes sowie gereinigte BSE-Proben bzw. entsprechende Negativkontrollen wurden von der VLA Weighbridge, Großbritannien (Dr. R. Jackmann) sowie der Bundesforschungsanstalt für Viruskrankheiten der Tiere, Insel Riems erhalten. Die Aufreinigung von PrP^{BSE} aus der *medulla oblongata* des Rinderhirns ohne Proteolyse entspricht prinzipiell der oben beschriebenen PrP^{Sc}-Aufreinigung aus Hamsterhirn. Unterschiede bestehen hinsichtlich der Sarkosylkonzentration bei der Homogenisierung des Gewebes. Bei Rindergewebe wurde im Gegensatz zum Hamstergewebe 4% Sarkosyl eingesetzt (nach Safar et al. (2002) Nat. Biotechnol. 20, 1147-1150), da sich bei geringeren Sarkosylkonzentrationen im Homogenat die Reinigungseffizienz und die PrP^{BSE}-Ausbeute erheblich verringerten. Zusätzlich wurde die Fällungsdauer auf vier Stunden reduziert. Es waren zwei Waschschritte erforderlich, um einen für FIDA-Messungen notwendigen Reinheitsgrad zu erreichen.

In Fig. 3 ist eine Western Blot-Analyse der einzelnen Reinigungsschritte der Fällung für eine Gewebeprobe eines BSE-infizierten Rindes und einer Kontrollprobe aus *medulla oblongata*-Gewebe abgebildet. Durch den Vergleich des mit Proteinase K behandelten BSE-Hirnhomogenats mit dem nicht behandelten (Fig. 3A) wird deutlich, dass die BSE-infizierte Probe nur einen geringen resistenten PrP^{BSE}-Anteil enthielt. Bei der Negativkontrolle (Abb. 3B) ist deutlich zu erkennen, dass PrP^{c} vollständig im Überstand verbleibt und das Pellet augenscheinlich PrP^{C}-frei ist. In der BSE-Probe waren im ersten Waschschritt geringe PrP-Mengen nachweisbar. Der Vergleich der Pelletfraktionen der BSE-Probe mit der Kontrollprobe lässt den Schluss zu, dass es sich bei dem PrP im Pellet der BSE-Probe um pathogenes PrP^{BSE} handelte. Die Tatsache, dass diese Probe nur einen geringen resistenten PrP^{BSE}-Anteil aufweist, lässt auch den Schluss zu, dass dieses PrP zum größten Teil sensitives PrP^{BSE} ist.

### Beispiel 3: Resupendierung der gefällten PrP-Aggregate

Auf das PrP^{Sc/BSE}-Pellet aus der NaPTA-Fällung wurden 200 µl PBS gegeben. Im Anschluss wurde das Pellet verschiedenen Ultraschallbedingungen ausgesetzt. Die Ultrabeschallbehandlung erfolgte drei mal zwei Sekunden mit Hilfe einer Ultraschall-Nadelsonde (Sonificator Labsonic U, Braun Dissel, Melsungen). Beim Einsatz der Nadelsonde wurde die Ultraschall-Sonde direkt in den Puffer eingetaucht.

### Beispiel 4: Entwicklung einer neuen Analysestrategie durch Immobilisierung pathologischer Prionenaggregate und Nachweis mittels 2D-FIDA-SPILA

Mittels eines SPILA (Single Particle Immuno-sorband Laserscanning Assay) werden gereinigte Prionenaggregate auf der Oberfläche eines Messchips mit Hilfe eines Fängermoleküls ("Capture-Molekül", etwa ein Antikörper) zu immobilisieren und anschließend zu detektieren (Fig. 4). Da die Immobilisierung eine Bewegung der Aggregate verhindert, kann die Oberfläche durch "Scannen" (i.e. durch Bewegung des konfokalen Volumenelements) nach Aggregaten abgesucht werden. Durch die Fixierung der Aggregate sollten zwei Vorteile genutzt werden. Zum einen sollte die Fixierung zur Reproduzierbarkeit der Ergebnisse beitragen und zum anderen sollten die Aggregate konzentriert werden. Weiterhin können Waschschritte durchgeführt und damit das Signal-Rauschverhältnis verbessert werden.

Eine Voraussetzung für den Nachweis immobilisierter einzelner Prionenpartikel mittels Messungen im FCS ist eine genaue Fokussierbarkeit des konfokalen Volumenelements auf die Höhe der zu scannende Fläche. Aus diesem Grund wurde in Zusammenarbeit mit dem Gerätehersteller Evotec Technologies (Hamburg) das FCS-Gerät um ein Piezoelement erweitert, das eine Höheneinstellung unabhängig von der Motorsteuerung mit einer Genauigkeit von 100 nm ermöglicht.

### Beispiel 5: Beschichtung von Glasoberflächen mit Fängermolekülproteinen

Um die Aggregate spezifisch auf der Glasoberfläche der verwendeten Messchips zu immobilisieren, muss diese zuvor mit einem Fängermolekül (z.B. einem Antikörper) beschichtet werden. Verfahren zur Beschichtung von Glasoberflächen mit Proteinen sind Fachleuten bekannt. Zur Evaluierung des Messansatzes wurde eine adhäsive Bindung des Fängermoleküls auf eine zuvor mit poly-D-Lysin aktivierte Oberfläche durchgeführt. Der gleiche Ansatz wurde auch mit kovalent gebundenen Antikörpern durchgeführt. Die weiteren Applikationen wurden jedoch mit adhäsiv beschichteten Fängermolekülen durchgeführt. Die Effizienz der Aktivierung der Glasoberfläche kann durch kurzes Abflammen der Glasoberfläche gesteigert werden. Zur Beschichtung wurden die 24-Well Assay-Chips (Evotec, Hamburg) mit Glasboden kurz mit Hilfe eines Bunsenbrenners abgeflammt. Anschließend wurden 20 µl poly-D-Lysin (10 µg/pl) in PBS in die Wells der Assay-Chips gegeben und eine Stunde bei 37°C (optional über Nacht bei 4°C) inkubiert. Anschließend wurde jeweils 1 µg Fängermolekül in PBS (in späteren Versuchen die Antikörper R1 (Inpro, USA) für Scrapie-Proben und Saf32 (Spibio, USA) für BSE-Proben) in die Wells gegeben und eine Stunde bei 37°C inkubiert. Im Anschluss wurden drei Waschschritte mit PBS durchgeführt und freie

Bindungsstellen durch eine einstündige Inkubation mit 5% (w/v) BSA geblockt.

### Beispiel 6: Immobilisierung von pathologischen PrP-Aggregaten

Zur spezifischen Immobilisierung der pathologischen PrP-Aggregate wurden PrP-spezifische Fängermoleküle verwendet. Zunächst wurde der Antikörper R1 als Fängermolekül für gereinigtes PrP^{Sc} aus Hirngewebe Scrapie-infizierter Hamster und entsprechender Negativkontrollen aus nicht infizierten Tieren eingesetzt. Hierfür wurden jeweils 1.25 x 10⁻³ gÄ gereinigte und mittels Ultraschall resuspendierte Proben in die mit R1 beschichteten Wells gegeben und für drei Stunden bei Raumtemperatur geschüttelt. Anschließend wurde dreimal für 10 Minuten mit TBST gewaschen. Um die immobilisierten Prionenpartikel mittels Fluoreszenzmessung nachweisen zu können, wurden die fluoreszenzmarkierten Antikörper R1 und D13 (Inpro, USA) in einer Konzentration von 0.1 µg/ml in TBST verwendet und für zwei Stunden unter Schütteln inkubiert. Abschließend wurde der Ansatz dreimal eine Stunde mit TBST gewaschen.

Um die Höhenbereiche über der Glasoberfläche des Analysechips zu evaluieren, in denen sich die immobilisierten Partikel befanden, wurden zunächst Messungen im FCS in z-Richtung durchgeführt werden. Anschließend wurden vergleichende Fluoreszenzmessungen mit PrP^{Sc}-Proben und Negativkontrollen durchgeführt. Die Fluoreszenzintensitätsverläufe lassen deutliche Unterschiede erkennen (Fig. 5). Bei Höhen von 5-20 µm über der Glasoberfläche, traten bei Scrapie-Proben viele Fluoreszenzintensitätspeaks auf, die in der Negativkontrolle nicht vorkamen. Diese sind auf die Bindung fluoreszenzmarkierter Antikörper an die pathologischen Prionenaggregate zurückzuführen.

Die Unterschiede zwischen Scrapie-infizierten und nicht infizierten Proben werden durch die Darstellung der 2D-FIDA-Daten noch deutlicher (Fig. 6). Die Bindung der beiden fluoreszenzmarkierten Antikörper an immobilisierte PrP-Aggregate führt zum Auftreten einer charakteristischen Diagonale der Fluoreszenzintensitäten im 2D-FIDA-Plot. Hierbei sind auf der x-Achse, die Fluoreszenz-Intensitäten des mit Alexa 488-markierten Antikörpers und auf der y-Achse die Fluoreszenz-Intensitäten des mit Alexa 633-markierten Antikörpers aufgetragen. Die Häufigkeiten des Auftretens der Fluoreszenzintensitäten sind in der z-Achse codiert (Graustufen). Da enorm starke Signale gemessen wurde, konnte diese charakteristische Kreuzkorrelationsdiagonale erst bei einer Höhe von 15 µm aufgelöst werden.

Das hier etablierte Protokoll zur Immobilisierung pathologischer Prionenaggregate und anschließender Markierung mit Hilfe fluoreszenzmarkierter Antikörper ist in Fig. 7 zusammengefasst.

### Beispiel 7: Optimierung der Messung immobilisierter Proteinaggregate mittels 2D-FIDA

Um Aggregate auf einer möglichst großen Fläche erfassen zu können, wurde der Messfokus nicht nur wie durch das FCS standardmäßig vorgegeben bestimmt ("gescannt"), sondern die zu erfassende Fläche erweitert. Hierzu wurden sieben nebeneinander liegende Flächen in einem Well des Assay-Chips (Fig. 8) gemessen. Eine komplette Messung der Bodenfläche eines Wells war bislang nicht möglich, da nur Chips mit runden Wells zur Verfügung standen und der Mittelpunkt des Wells manuell eingestellt werden musste, so dass ein "Sicherheitsabstand" nötig war, um zu verhindern einen Well während der Messung zu verlassen. Wie in den einzelnen Bestimmungen zu sehen, war die Aggregatverteilung über die verschiedenen Flächen erwartungsgemäß nicht gleich. Durch Summierung der Flächen ergibt sich jedoch eine repräsentative Beschreibung der Probe.

Mit Hilfe der etablierten Immobilisierungs- und Messprobe wurde ein "Höhenscan" durch eine Scrapie-Probe und eine Negativkontrolle durchgeführt. Die Messungen wurden ausgewertet, indem die Häufigkeiten der Fluoreszenzintensitäten über 15 µm summiert wurden. Die Ergebnisse der 2D-FIDA-Messungen zeigen, dass zwischen der PrP^{Sc}-Probe und der Negativkontrolle ein signifikanter Unterschied messbar ist (Fig. 9).

### Beispiel 8: 2D-FIDA Reihenmessungen von PrP^{Sc} und PrP^{BSE}

Um die Messprobe hinsichtlich einer diagnostischen Anwendbarkeit zu evaluieren, wurden Messreihen mit Proben von vier verschiedenen infizierten und vier nicht infizierten Tieren als Kontrollen durchgeführt. Dazu wurden Hirngewebeproben Scrapie-infizierter Hamster und Negativkontrollen mittels NaPTA-Fällung gereinigt und 1.25 x 10⁻³ gÄ nach Resuspension mittels 3 x 2 Sekunden Ultraschall mit dem Antikörper R1 als Fängermolekül auf den Assay-Chips immobilisiert. Zur Detektion wurden je 0.1 µg/ml fluoreszenzmarkierter Antikörper R1 und D13 als Sonden eingesetzt. Nach drei einstündigen Waschschritten mit TBST wurden Fluoreszenzmessungen im FCS von sieben einzelnen Flächen durchgeführt. Die Messungen wurden in verschiedenen Höhen über dem Glasboden durchgeführt. Die Ergebnisse (Fig. 10) zeigen, dass in den Höhenbereichen von 5 µm bis 25 µm ein signifikanter Unterschied zwischen mit Scrapie infizierten und nicht infizierten Hamstern erkennbar ist. Die Messhöhe zwischen 10 µm und 20 µm stellte sich dabei als geeignete Messhöhe dar, da oberhalb dieser Distanz die positiven Signale erheblich abfallen und unterhalb 10 µm der Messhintergrund durch unspezifische Antikörperbindung und freie Farbstoffmoleküle erhöht ist.

Zusammenfassend ließ sich die Aussage treffen, dass im Hamstermodell Scrapie-infizierte von nicht infizierten Hamstern signifikant unterschieden werden konnten und im Vergleich zum Messansatz in Suspension nicht nur die Unterschiede zwischen positiven und negativen Proben eindeutiger waren, sondern vor allem die Standardabweichungen enorm reduziert werden konnten.

Das System wurde anschließend für BSE-Proben adaptiert. Hierzu wurde als Fängermolekül der Antikörper 12F10 (Firma Spibio) eingesetzt. Alle anderen Schritte erfolgen analog zu denen des oben beschriebenen Scrapie-Systems. Als Sonden zur Detektion wurden die fluoreszenzmarkierten Antikörper 12F10 und Saf32 (Spibio, USA) eingesetzt.

Die Ergebnisse für vier BSE-infizierte Tiere und vier Negativkontrollen sind in Fig. 11 dargestellt. Die Messung zeigt, dass im Bereich von 10 µm bis 25 µm Abstand zur Chipoberfläche in allen Fällen ein signifikanter Unterschied zwischen BSE-infizierten und nicht infizierten Tieren zu erkennen war. Der Bereich von 10 µm bis 15 µm war der am besten geeignete Messbereich, da ab 20 µm das positive Signal in einigen Proben stark absank.

Durch diese Messreihen konnte gezeigt werden, dass das SPILA für einen diagnostischen Assay zur Detektion von BSE und für Scrapie geeignet ist.

### Beispiel 9: 2D-FIDA-Messungen immobilisierter BSE-Liqourproben vom Rind

Das erfindungsgemäße Verfahren wurde ebenfalls auf den Einsatz von Liquor als Probenmaterial adaptiert.

Dazu wurde eine Reihenmessung mit fünf Proben durchgeführt. Die Glasoberfläche der Assay-Chips wurde wie in Beispiel 5 beschrieben mit dem entsprechenden Fängermolekül (hier Saf32) beschichtet. Dann wurden 20µl reiner Liqour von terminal an BSE erkrankten Rindern sowie Liqour von gesunden Rindern als Negativkontrollen eingesetzt. Der pure Liqour wurde vor dem Einsatz dreimal für zwei Sekunden ultrabeschallt, dann in die beschichteten Assay-Chips gegeben und zur Bindung für zwei Stunden bei Raumtemperatur geschüttelt und anschließend bei 4°C über Nacht inkubiert. Die weitere Behandlung und Messung erfolgte nach dem bereits in den Beispielen 6-8 beschriebenen Protokoll. Als Detektionssonden wurden die fluoreszenzmarkierten Antikörper Saf32 (Firma Spibio) und 12F10 (Firma Spibio) eingesetzt. Das Ergebnis der Messungen in einer Höhe von fünf µm über dem Boden der Assay-Chips ist in Fig. 12 dargestellt.

In dieser Messreihe konnten vier BSE-Proben von den Negativkontrollen unterschieden werden. Nur eine BSE-Probe wies ein Signal auf, das nicht höher war als der Hintergrund.

Um den Ansatz zu optimieren, wurden zunächst mögliche Fängermolekül/Detektionssonden Kombinationen getestet. Dazu wurden als Fängermoleküle die Antikörper Saf32 (Firma Spibio) und D18 (Firma Inpro) mit den fluoreszenzmarkierten Detektionssonden Antikörper Saf32 (Firma Spibio), 12F10 (Firma Spibio) und D18 (Firma Inpro) kombiniert. Die Messansätze wurden mit zwei BSE-Liqourproben und zwei Negativkontrollen im Doppelansatz durchgeführt. Der Messansatz wurde wie bei der ersten Messreihe beschrieben durchgeführt. Das Ergebnis der Messreihe in einer Höhe von fünf µm über dem Boden der Assay-Chips ist in Fig. 13 dargestellt.

Die Messreihe zeigt, dass mit allen Kombinationen eine Unterscheidung der BSE-Proben und der Negativkontrollen möglich ist. Die Kombination von D18 als Fängermolekül und Saf32/12F10 als Detektionssonden weist die aussichtsreichsten Ergebnisse auf, da die Schwankungsbreite zwischen den Proben einer Gruppe am geringsten ist und eine klare Unterscheidung erfolgen kann.

Das erfindungsgemäße Verfahren ist daher zur Diagnostik von BSE anhand einer Liquor-Messprobe geeignet.

## Patentansprüche

1. In-vitro Verfahren zur selektiven Bestimmung der Gegenwart und/oder der Menge pathologischer Proteinablagerungen des PrP^{SC} in einer Messprobe, die aus einer Körperflüssigkeit aus der Gruppe ausgewählt aus Cerebrospinalflüssigkeit, Lymphflüssigkeit, Blut, Urin und Sputum oder einem Gewebe stammt und die pathologischen Proteinablagerungen des PrP^{SC} mit Scrapie und boviner spongiformer Encephalopathie assoziiert sind, wobei das Verfahren umfasst:
(a) Immobilisieren eines Fängermoleküls, das spezifische Bindungsaffinität für Teilstrukturen der zu bestimmenden PrP^{SC} Proteinablagerungen aufweist, auf einer Oberfläche;
(b) In-Kontakt-bringen des immobilisierten Fängermoleküls mit der Messprobe, von der vermutet wird, dass sie pathologische Proteinablagerungen enthält;
(c) Inkubieren des Ansatzes, sodass die Bildung eines Komplexes aus dem immobilisierten Fängermolekül und den Teilstrukturen der zu bestimmenden PrP^{SC} Proteinablagerungen ermöglicht wird;
(d) In-Kontakt-bringen des Komplexes mit zwei detektierbaren Einheiten, die spezifische Bindungsaffinität für die Teilstrukturen der zu bestimmenden PrP^{SC} Proteinablagerungen aufweisen, die simultan an den Komplex binden und die optisch detektierbare Signale erzeugen, wobei die zwei detektierbaren Einheiten ein mittels spektroskopischer Verfahren detektierbares Signal erzeugen; und
(e) Detektieren der Komplexbildung durch Messung der vonden zwei detektierbaren Einheiten insgesamt erzeugten Signale mittels Fluoreszenzkorrelationsspektroskopie (FCS) kombiniert mit Kreuzkorrelation und Single Particle Immunosorbent Laserscanning Assay;
(f) Messung der von den zwei detektierbaren Einheiten insgesamt erzeugten Signale durch Abrastern der Oberfläche.

2. Verfahren gemäss Anspruch 1, wobei die Teilstrukturen der zu bestimmenden pathologischen PrP^{SC} Proteinablagerungen monomere oder oligomere Einheiten der Proteinablagerungen oder Fragmente davon umfassen.

3. Verfahren gemäss einem der Ansprüche 1 oder 2, das ferner die Aktivierung der Oberfläche vor dem Immobilisieren des Fängermoleküls umfasst.

4. Verfahren gemäss einem der Ansprüche 1 bis 3, wobei das Fängermolekül aus der Gruppe ausgewählt wird, die aus monoklonalen Antikörpern, polyklonalen Antikörpern und Antikörperfragmenten besteht.

5. Verfahren gemäss einem der Ansprüche 1 bis 3, wobei das Fängermolekül aus Teilstrukturen pathologischer Proteinablagerungen besteht.

6. Verfahren gemäss Anspruch 1, wobei das Gewebe Hirngewebe ist.

7. Verfahren gemäss einem der Ansprüche 1 bis 6, wobei die Messprobe vor dem In-Kontakt-bringen mit dem immobilisierten Fängermolekül gereinigt wird.

8. Verfahren gemäss Anspruch 7, wobei die Reinigung eine Phosphowolframat-Fällung ohne Zugabe einer Proteinase umfasst.

9. Verfahren gemäss Anspruch 1, wobei die von den zwei oder mehr detektierbaren Einheiten optisch erzeugten Einzelsignale durch Koinzidenzmessung bestimmt werden.

10. Verfahren gemäss einem der Ansprüche 1 bis 9, wobei die zwei detektierbaren Einheiten ein Protein oder ein Polypeptid umfassen.

11. Verfahren gemäss Anspruch 10, wobei das Protein oder Polypeptid aus der Gruppe ausgewählt wird, die aus monoklonalen Antikörpern, polyklonalen Antikörpern und Antikörperfragmenten besteht.

12. Verfahren gemäss einem der Ansprüche 1 bis 11, wobei das von den zwei detektierbaren Einheiten erzeugte optisch detektierbare Signal aus der Gruppe ausgewählt wird, die aus Fluoreszenzemission, Chemilumineszenzemission und Biolumineszenzemission besteht.

13. Verfahren gemäss Anspruch 12, wobei zur Auswertung der Ergebnisse eine Fluoreszenzintensitätsdistributionsanalyse durchgeführt wird.

14. Verfahren gemäss Anspruch 1 bis 13, wobei eine Mehrzahl nebeneinander liegender Teilbereiche der Oberfläche abgerastert wird, und die Einzelwerte anschliessend addiert werden.

## Claims

1. An in-vitro process for the selective determination of the presence and/or amount of pathological protein deposits of PrP^{Sc} in a sample to be measured, which is derived from a body fluid selected from the group consisting of cerebrospinal fluid, lymph, blood, urine and sputum, or from a tissue, said pathological protein deposits of PrP^{Sc} being associated with scrapie and bovine spongiform encephalopathy, the process comprising:
(a) immobilizing on a surface a capture molecule having specific binding affinity for substructures of the PrP^{Sc} protein deposits to be determined;
(b) contacting the immobilized capture molecule with the sample to be measured, which is suspected of containing pathological protein deposits;
(c) incubating the preparation to allow a complex to be formed from the immobilized capture molecule and said substructures of the PrP^{Sc} protein deposits to be determined;
(d) contacting the resulting complex with two detectable units having specific binding affinity for said substructures of the PrP^{Sc} protein deposits to be determined, binding simultaneously to the complex and producing optically detectable signals, wherein said two detectable units produce a signal detectable by means of spectroscopic methods; and
(e) detecting the complex formation by measuring the overall signals produced by said two detectable units by means of fluorescence correlation spectroscopy (FCS) in combination with cross-correlation and single particle immunosorbent laser-scanning assay;
(f) measuring the overall signals produced by said two detectable units by scanning the surface.

2. The process according to claim 1, wherein said substructures of the pathological PrP^{Sc} protein deposits to be determined comprise monomeric or oligomeric units of the protein deposits or fragments thereof.

3. The process according to either of claims 1 or 2, which further comprises the activation of the surface before the capture molecule is immobilized thereon.

4. The process according to any of claims 1 to 3, wherein said capture molecule is selected from the group consisting of monoclonal antibodies, polyclonal antibodies and antibody fragments.

5. The process according to any of claims 1 to 3, wherein said capture molecule consists of substructures of pathological protein deposits.

6. The process according to claim 1, wherein said tissue is brain tissue.

7. The process according to any of claims 1 to 6, wherein said sample to be measured is purified before being contacted with the immobilized capture molecule.

8. The process according to claim 7, wherein said purification includes phosphotungstate precipitation without adding a proteinase.

9. The process according to claim 1, wherein the individual signals produced by the two or more detectable units are determined by coincidence measurement.

10. The process according to any of claims 1 to 9, wherein said two detectable units comprise a protein or polypeptide.

11. The process according to claim 10, wherein said protein or polypeptide is selected from the group consisting of monoclonal antibodies, polyclonal antibodies and antibody fragments.

12. The process according to any of claims 1 to 11, wherein the optically detectable signal produced by said two detectable units is selected from the group consisting of fluorescence emission, chemiluminescence emission and bioluminescence emission.

13. The process according to claim 12, wherein a fluorescence intensity distribution analysis is performed for evaluating the results.

14. The process according to claims 1 to 13, wherein a plurality of adjacent subareas of the surface are scanned, and the individual values are subsequently added.

## Revendications

1. Procédé in-vitro pour la détermination sélective de la présence et/ou de la quantité de dépôts protéiques pathologiques de la PrP^{SC} dans un échantillon de mesure qui provient d'un liquide corporel issu du groupe sélectionné parmi du liquide cérébrospinal, de la lymphe, du sang, de l'urine et du crachat ou d'un tissu, et des dépôts protéiques pathologiques de la PrP^{SC} sont associés à la tremblante et à l'encéphalopathie spongiforme bovine, le procédé comprenant :
(a) l'immobilisation sur une surface d'une molécule de capture qui présente une affinité de liaison spécifique pour des structures partielles des dépôts protéiques PrP^{SC} à déterminer ;
(b) la mise en contact de la molécule de capture immobilisée avec l'échantillon de mesure dont on suppose qu'il contient des dépôts protéiques pathologiques ;
(c) la mise en incubation de la préparation, de sorte que la formation d'un complexe à partir de la molécule de capture et des structures partielles des dépôts protéiques PrP^{SC} à déterminer est rendue possible ;
(d) la mise en contact du complexe avec deux unités détectables qui présentent une affinité de liaison spécifique pour les structures partielles des dépôts protéiques PrP^{SC} à déterminer, qui se lient simultanément au complexe et qui produisent des signaux optiquement détectables, les deux unités détectables produisant un signal détectable au moyen de procédés spectroscopiques ; et
(e) la détection de la formation de complexe par la mesure des signaux produits au total par les deux unités détectables au moyen de la spectroscopie de corrélation de fluorescence (FCS) combinée à la corrélation croisée et au Single Particle Immunosorbent Laserscanning Assay ;
(f) la mesure des signaux produits au total par les deux unités détectables par le balayage de la surface.

2. Procédé selon la revendication 1, les structures partielles des dépôts protéiques PrP^{SC} pathologiques à déterminer comprenant des unités monomères ou oligomères des dépôts protéiques ou de fragments de ceux-ci.

3. Procédé selon une des revendications 1 ou 2, qui comprend en outre l'activation de la surface avant l'immobilisation de la molécule de capture.

4. Procédé selon une des revendications 1 à 3, la molécule de capture étant sélectionnée dans le groupe composé d'anticorps monoclonaux, d'anticorps polyclonaux et de fragments d'anticorps.

5. Procédé selon une des revendications 1 à 3, la molécule de capture se composant de structures partielles de dépôts protéiques pathologiques.

6. Procédé selon la revendication 1, le tissu étant du tissu cérébral.

7. Procédé selon une des revendications 1 à 6, l'échantillon de mesure étant nettoyé avant la mise en contact avec la molécule de capture immobilisée.

8. Procédé selon la revendication 7, le nettoyage comprenant une précipitation de phosphotungstate sans ajout de protéinase.

9. Procédé selon la revendication 1, les signaux individuels produits optiquement par les deux unités détectables ou plus étant déterminés par mesure en coïncidence.

10. Procédé selon une des revendications 1 à 9, les deux unités détectables comprenant une protéine ou un polypeptide.

11. Procédé selon la revendication 10, la protéine ou le polypeptide étant sélectionné(e) dans le groupe composé d'anticorps monoclonaux, d'anticorps polyclonaux et de fragments d'anticorps.

12. Procédé selon une des revendications 1 à 11, le signal détectable optiquement produit par les deux unités détectables étant sélectionné dans le groupe composé de l'émission de fluorescence, de l'émission de chimioluminescence et de l'émission de bioluminescence.

13. Procédé selon la revendication 12, une analyse de distribution de l'intensité de fluorescence étant effectuée pour l'analyse des résultats.

14. Procédé selon les revendications 1 à 13, une multiplicité de zones partielles juxtaposées de la surface étant balayées, et les valeurs individuelles étant ensuite additionnées.
